# EUROPEAN PATENT APPLICATION

(11) **EP 4 596 683 A1**
(43) Date of publication of application: **06.08.2025**
(21) Application number: 23873264.8
(22) Date of filing: 27.09.2023
(51) Int. Cl.: C12N 5/09, A01N 1/02

(54) **METHOD FOR PREPARING ORGANOID**

(30) Priority: 27.09.2022 KR 20220122551
(71) Applicant: CHUNGNAM NATIONAL UNIVERSITY HOSPITAL, Daejeon 35015 (KR)
(72) Inventor: CHUNG, Chaeuk, Daejeon 35248 (KR); PARK, Dongil, Daejeon 34933 (KR)
(74) Representative: Gille Hrabal Partnerschaftsgesellschaft mbB Patentanwälte
(86) International application number: PCT/KR2023/015128
(87) International publication number: WO 2024/072157

(57) **Abstract**

The present invention relates to a novel method for producing organoids that resolves the problem of low organoid yield caused by enzymatic processing by substituting enzymatic cell dissociation with mechanical dissociation in conventional organoid production, more specifically, to a method for producing organoids comprising: (A) rapidly freezing biological tissue; (B) thawing the rapidly frozen specimen; (C) mechanically dissociating the thawed specimen to obtain dissociated cells; and (D) producing organoids using the dissociated cells.

## Description

### Technical Field

The present invention relates to a method for producing organoids from biological tissue, and more specifically, to a novel method for producing organoids that resolves the problem of low organoid yield caused by enzymatic processing by substituting enzymatic cell dissociation with mechanical dissociation in conventional organoid production.

### Background Art

An organoid is an organ mimic produced by growing cells in three-dimensional culture. Organoids are cell line-like experimental models that have the advantage of being capable of long-term culture and cryopreservation, as well as being easy to manipulate and observe. Furthermore, they are experimental models that enable the study of physiological phenomena at a higher level than cells by maintaining the original characteristics of cells and reproducing cellular hierarchical and histological structures that could only be observed in vivo.

Organoids can be applied in two aspects: as disease models that leverage their similarity to real organs, and as regenerative therapeutics that leverage their tissue regenerative capabilities.

Organoid-based disease models are human organ models that can be produced using human tissue, providing results more similar to in vivo conditions than animal experiments or monolayer culture experiments. Organoids enable research on developmental and regenerative mechanisms through normal organ modeling, as well as on disease mechanisms and drug development through organ disease modeling, while cancer organoids, in particular, enable applications across most tumor research fields, from tumorigenesis mechanisms to precision medicine and anticancer drug development. The second application, organoid-based regenerative therapeutics, can be a fundamental therapeutic agent that can repair tissue damage by directly implanting organoids into damaged tissues.

To date, various types of organoids have been produced, including salivary glands, liver, lacrimal glands, bile ducts, thyroid glands, hair follicles, pancreas, stomach, and endometrium, but organoid culture success rates are known to vary considerably depending on the organ and the size of the target cell sample. For example, when surgical tissue is used as the source, the culture success rate is high, but when organoids are produced from small samples such as biopsy specimens, the culture success rate is significantly lower (Cells. 2021 Nov 4;10(11):3012). Particularly in tumor research, organoids have a major limitation in that they cannot mimic the actual tumor microenvironment (Nat. Commun.2019,10, 3991). Additionally, in conventional methods for producing cancer cell organoids, cancer tissue is first finely cut (mechanical dissociation) with scissors or a knife, followed by enzymatic treatment with enzymes such as dispase to cleave cell surface fibronectin, collagen, etc., thereby dissociating cells and decomposing the cancer tissue's connective tissue. While this conventional method effectively dissociates tissues into single cells, some cells may die due to enzyme cytotoxicity, and even surviving cells may be in poor condition and often fail to divide properly.

Despite significant improvements in treatment outcomes over the past decade due to the development of innovative therapies such as targeted agents and immunotherapies, lung cancer remains the most common cause of cancer-related mortality, with an overall five-year survival rate of merely 32.5%. Accordingly, extensive research utilizing organoids is being conducted to elucidate lung cancer pathogenesis mechanisms, screen novel therapeutic agents, and develop personalized cancer treatments. However, attempts to produce lung cancer organoids from tissue biopsy specimens from patients with advanced and metastatic lung cancer have shown notably low success rates of approximately 10-20% (Cell Rep.2020,31, 10758 / NPJ Precis. Oncol.2021,5, 29).

US 2021-0087532 A1 discloses a technique for producing organoids from cryopreserved tissue without time constraints, comprising the steps of: thawing cryopreserved tissue in suspension in a preservation medium to obtain isolated cells; washing the cryopreservation medium; and treating the cryopreserved tissue with an enzyme (Dispase) to dissociate epithelial cells at the base of the mesenchyme. This document reports that it is possible to produce organoids from long-term cryopreserved tissue, although they are smaller and less structured (crypts) than organoids from fresh tissue that has not undergone the cryopreservation process.

### Detailed Description of the Invention

### Technical Problem

An object of the present invention is to provide a method for readily producing organoids from biological tissue, particularly biopsy tissue.

Another object of the present invention is to provide a method for producing organoids without enzymatic treatment, thereby eliminating potential adverse effects associated with enzymatic processing.

### Technical Solution

To achieve the aforementioned objectives,
the present invention relates to a method for producing organoids comprising: (A) rapidly freezing biological tissue; (B) thawing the rapidly frozen specimen; (C) mechanically dissociating the thawed specimen to obtain dissociated cells; and (D) producing organoids using the dissociated cells. In step (A), the entire or local region of biological tissue isolated by surgery or biopsy may be locally rapidly frozen, or it may be locally rapidly frozen before isolation during surgery or biopsy. In the present invention, there may be a temporal separation between steps (A) and (B). That is, the 'frozen specimen' in the present invention may be obtained immediately from living tissue or may be tissue that has been obtained and cryopreserved.

In the present invention, step (A) may include a sub-step of contacting a cryoprobe tip to a predetermined site for obtaining dissociated cells from biological tissue for local freezing, and a sub-step of isolating only the locally frozen region from the biological tissue. Preferably, the cryoprobe tip temperature is -40 to -130°C and the contact time is 3-5 seconds. Detailed descriptions of cryoprobe types, handling temperatures, etc. are omitted as these are widely known in the technical field to which the present invention belongs. By utilizing a cryoprobe, the present invention enables acquisition of cell clusters from local regions of interest within biological tissue, thereby maximizing cell homogeneity in the obtained specimens. For example, it becomes possible to isolate and obtain high-purity specimens from only the lesion sites where cancer cells are expressed within biological tissue.

In the present invention, the biological tissue may be isolated by percutaneous or endoscopic biopsy (cryobiopsy or forceps biopsy), but it is not excluded that it may be obtained by surgical means. In the present invention, said biological tissue may be, but is not limited to, cancer tissue, more specifically lung cancer tissue. The lung cancer tissue is preferably, but not exclusively, obtained by a non-surgical biopsy procedure. In the present invention, the lung cancer tissue may be obtained by a non-surgical frozen biopsy procedure.

One of the best methods to avoid controversy regarding cancer cell purity is to utilize tissue with minimal normal cells when initially establishing cancer organoids, specifically by producing cancer organoids from malignant pleural effusion cancer cells or tissue obtained from metastatic lesions, which lack normal cells. However, for example, only 8-12% of non-small cell lung cancer patients present with malignant pleural effusion at diagnosis, and cancer cells from metastatic lesions may exhibit different characteristics compared to those from primary tumor sites. Therefore, non-surgical tissue specimens obtained through selective cancer lesion sampling methods such as percutaneous biopsy, endoscopy, and endoscopic ultrasound serve as excellent source material for producing high-purity cancer organoids.

### Effects of the Invention

As shown above, according to the present invention, organoids can be produced using cells obtained by physical dissociation rather than 'enzymatic dissociation', thereby enabling organoid production without the adverse effects associated with 'enzymatic treatment'.

Furthermore, the present invention enables simpler and more efficient production of desired organoids as it eliminates the need for 'enzymatic dissociation'.

Additionally, the present invention facilitates the production of organoids not only from surgically obtained specimens but also from biopsy-derived biological tissue, thereby enabling the production of lung cancer organoids from lung cancer biopsy specimens of patients with stage 3 and 4 lung cancer, which was previously nearly impossible, and allowing for personalized anticancer treatment and screening of anticancer drugs using the organoids.

### Brief Description of the Drawings

FIGS. 1, 2 are photographs showing the preprocessing of surgical tissue and small biopsy tissue, respectively.
FIG. 3 is a photograph showing the process of producing organoids in an embodiment of the present invention.
FIG. 4 is a conceptual diagram showing the structure of the cryoprobe used in the embodiment and a photograph showing the rapid freezing process.
FIGS. 5 to 7 are various photographs of organoids produced according to the present invention.
FIGS. 8, 9 are Holotomography photographs of organoids produced according to the present invention.
FIG. 10 is diagrams of Single cell RNA sequencing results of organoids according to the embodiment.
FIG. 11 is UMAPs showing the types and states of organoid cells according to the embodiment.
FIG. 12 is a conceptual diagram showing the drug sensitivity test situation of organoids according to the embodiment.
FIGS. 13 and 14 are photographs and graphs, respectively, showing the viability and survival rates of organoids against drugs according to the enbodiment.

### Best Mode for Carrying Out the Invention

As described above, the present invention is a tissue specimen dissociation method for the successful culture of biopsy specimen-derived organoids, which have conventionally had low culture success rates, through the process of rapid freezing and thawing of tissue. Specifically, the present invention improves the success rate of organoid culture by eliminating the conventional enzymatic dissociation process and applying rapid freezing and thawing to the specimen tissue. To date, no confirmed cases of using rapid freezing and thawing methods in organoid culture have been reported.

As confirmed in the following examples, when tissue was rapidly frozen-thawed and physically dissociated into single cells without enzyme treatment before inducing organoids, tissue organoids were produced larger and faster compared to when using the conventional enzymatic dissociation method.

The organoid production method according to the present invention has a wide range of applications. For example, it can be utilized in the treatment of lung cancer as follows.

Recently, when lung cancer progresses after anti-cancer treatment, re-biopsy is performed to identify new gene mutations or drug resistance mechanisms to select the next treatment drug. A typical example is starting Osimertinib treatment when EGFR T790M mutation is confirmed through re-biopsy in cases where disease progresses after EGFR-TKI treatment. Additionally, it is common practice to perform tissue biopsy on suspicious lesions to confirm recurrence when recurrence is suspected in various sites after surgery or chemotherapy/radiation therapy. If lung cancer organoids can be produced using these tissue biopsy specimens, it could greatly help in selecting the most appropriate treatment drugs for patients at the time of lung cancer progression or recurrence.

Furthermore, lung cancer organoids created from various locations and at different time points enable more in-depth research into the heterogeneity of cancer cells from different sites, drug sensitivity patterns, and characteristics of drug-resistant cancer cells.

Moreover, by establishing a lung cancer organoid cluster comprising organoids created from various sites and time points from a single patient, it becomes possible to conduct various research by selecting the optimal lung cancer organoid group needed for the study, not only for personalized patient treatment but also for candidate drug screening.

The present invention will now be described in more detail with reference to the accompanying drawings and embodiments. However, these drawings and embodiments are illustrative only to facilitate the explanation of the content and scope of the technical concept of the invention, and do not limit or change the technical scope of the invention. Based on these embodiments, it will be apparent to those skilled in the art that various modifications and changes are possible within the scope of the technical concept of the invention.

### Embodiments

### 1. Acquisition of Lung Cancer Tissue

Specimens were obtained with patient consent from: ① Patients who underwent bronchoscopy, lung biopsy, or surgery for pulmonary nodules or lung cancer ② Patients who underwent bronchoscopy, lung biopsy, or surgery for interstitial lung disease or pneumothorax ③ Male and female patients aged 19 or older with stage 1-4 lung cancer (non-small cell lung cancer, small cell lung cancer). Lung cancer tissues isolated from patients were stored in DMEM/F12 medium (Gibco, Waltham, MA, USA) containing 1% penicillin and streptomycin, and subjected to the following procedures within 12 hours.

### 2. Dissociation of Lung Cancer Tissue

### (1) Preprocessing

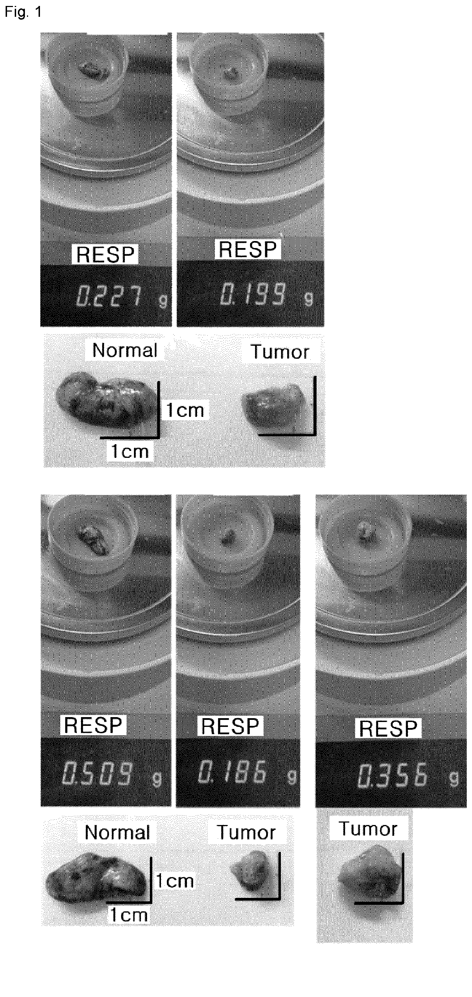

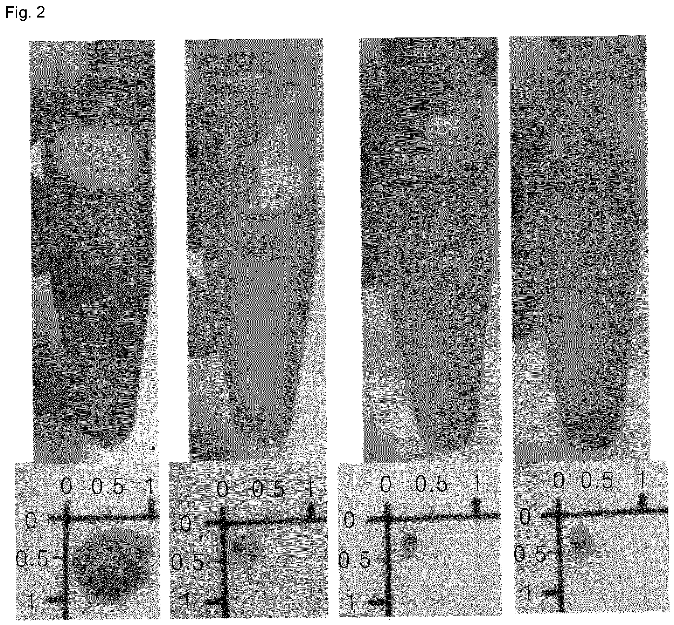

After isolating only the lesion area of the obtained lung cancer tissue, it was cut evenly into two parts and used as specimens for the example according to the present invention (using the freeze-thaw dissociation method) and for the comparative example using the conventional enzymatic treatment method. Photographs showing the preprocessing of surgical tissue and small biopsy tissue are shown in FIGS. 1 and 2, respectively. Separately, lung organoids (organoids derived from normal lung cells) were produced using cancer-free portions of the resected lung.

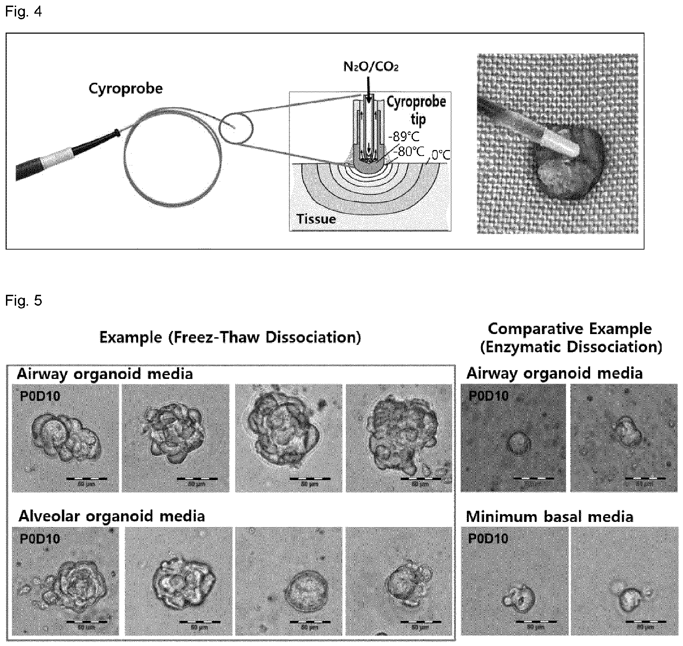

On the other hand, there was no significant difference in organoid production between samples dissociated by the freeze-thaw method after isolating only the lesion area of lung cancer tissue in several preliminary experiments (Example Type 1: surgical tissue acquisition → freezing → thawing → mechanical dissociation) and samples directly obtained through cryobiopsy in a frozen state, then thawed and dissociated (Example Type 2: endoscopic cryobiopsy → thawing → mechanical dissociation). Therefore, Type 1 or Type 2 was applied depending on the situation. For example, the examples in FIG. 5 to 8 are Type 1, while the remaining examples are Type 2.

### (2) Dissociation of specimens for Examples

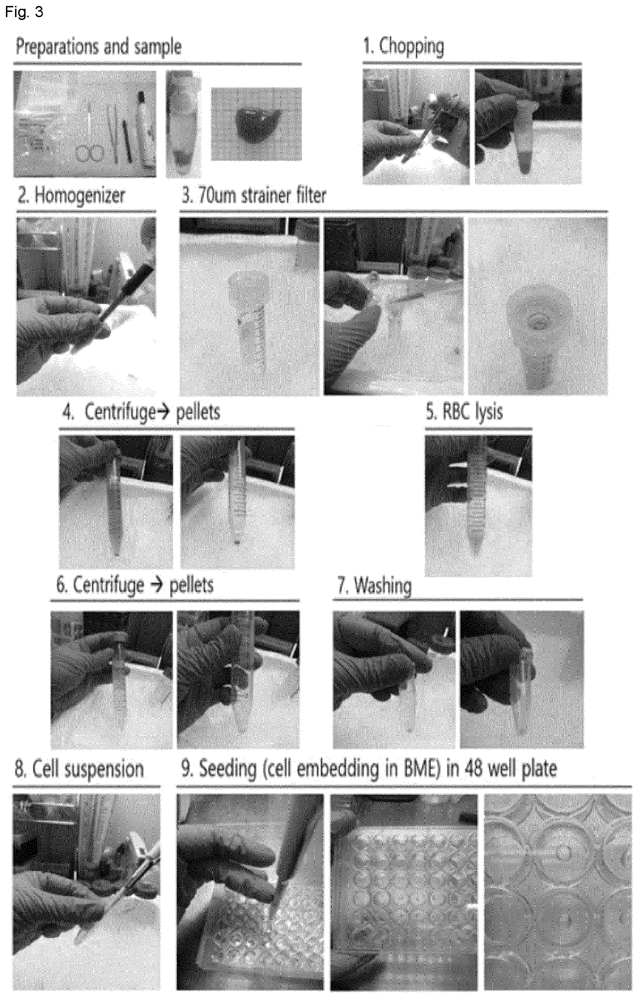

Freeze-thaw dissociation was performed as follows. The process of mechanical dissociation after thawing and producing organoids from it is shown in FIG. 3.
① The lung cancer tissue lesion was rapidly frozen by applying a cryoprobe tip with a tip temperature of approximately -90°C for 3 to 5 seconds, followed by automatic thawing (automatic thawing occurred when the tip was removed). In this example, lung cancer tissue isolated from a human patient was used, but preliminary experiments showed similar results were obtained when tissue acquired directly by cryobiopsy from human patients was processed through subsequent steps. A conceptual diagram showing the structure of the cryoprobe (1.9 mm cryoprobe, CRYO2; Erbe Elektromedizin GmbH, Tubingen, Germany) used in this example and a photograph demonstrating the rapid freezing process are shown in FIG. 4. Various other methods may be employed for rapid freezing and thawing.
② The specimen was treated with 1 ml of DMEM/F12 (Gibco; 11330032) containing 2% antibiotics (Sigma; A5955), finely cut with scissors, and further fragmented using a Handy homogenizer.
③ The tissue fragments were filtered using a 70 µm cell strainer, and the cells were collected while being washed with DMEM/F12+ (containing antibiotics).
④ The specimen was centrifuged at 300×g for 5 minutes at 4°C and the supernatant was removed. If the supernatant appears red, it indicates the presence of red blood cells in the pellet, requiring red blood cell lysis.
   For red blood cell lysis, 1 ml of 1× RBC lysis buffer (1 ml of 10× lysis buffer + 9 ml of D.D.W.) was added and tapped, and after 5 minutes, RBC lysis was stopped using DPBS (without calcium and magnesium; Dulbecco's phosphate-buffered saline, Thermo Fisher, USA) containing 10% FBS.
⑤ The specimen was centrifuged at 300×g for 5 minutes at 4°C, the supernatant was removed, and the pellet was washed with 1 ml of DPBS. The obtained cells were then counted.

### (3) Specimen Dissociation for Comparative Example

'Enzymatic dissociation' was performed following the same process as (1) above, except that in step ①, 1 ml of Dissociation Buffer with the composition shown in the table below was added to the lung cancer tissue lesion, finely cut with scissors, and subjected to enzyme digestion (DNase (sigma; AMPD1), liberase) for 30 minutes at 37°C.

| | |
|---|---|
| DMEM | Gibco; 11330032, USA |
| DNase, Final Conc. 10 mg/ml | Sigma; AMPD1, USA |
| Collagenase/dispase, Final Conc. 1 mg/ml | Roche: 10269638001 stock 100 mg/ml |
| Antibiotics Final Conc. 2% | Sigma; A5955 USA |

### 3. Cell Culture and Organoid Production

Cells were cultured and organoids were produced according to the following scheme (see FIG. 3).
① 20µl Matrigel (Corning, 354230) was added to the cell pellets (adjusted to contain approximately 1000 cells) and mixed without creating bubbles.
② 20µl of the mixture was dropped into a 37°C prewarmed 48-well plate without creating bubbles.
③ The plate lid was closed, inverted and incubated at 37°C for 5 minutes to form a dome.
④ The plate was removed, returned to its original position, and 200µl of airway organoid media (composition shown in the table below) was added along the well side without touching the Matrigel.

| Human Lung airway (basal & club) cell organoid (2019 Norman Sachs et al) | |
|---|---|
| Composition | Conc. |
| AdDMEM/F12 (Gibco, USA) | |
| HEPES (Gibco, USA) | 10 mM |
| Glutamax (Gibco, USA) | 1X |
| P/S (penicillin, streptomycin) (Gibco, USA) | 1X |
| R-spondin1 (Sigma, USA) | 500 ng/ml |
| hFGF7 (Sigma, USA) | 25 ng/ml |
| hNoggin (Sigma, USA) | 100 ng/ml |
| hFGF10 (Sigma, USA) | 100 ng/ml |
| B27 supplement (Sigma, USA) | 1X |
| N-acetylcysteine (Sigma, USA) | 1.25 mM |
| Primocin (Sigma, USA) | 50 µg/ml |
| SB431542 (Sigma, USA) | 500 nM |
| Nicotinamide (Sigma, USA) | 5 mM |
| A83-01 (Sigma, USA) | 500 µM |
| Y27632 (Sigma, USA) | 5 µM |

⑤ Cultured under 5% carbon dioxide, 37°C condition. The medium was changed every 4 days, and because different organoids grew at different rates, the organoids were monitored under a microscope during culture, and when the number of organoids increased and they grew larger, they were subjected to passage every 1-3 weeks as follows: Organoids (2-3 weeks after culture initiation) were harvested in DPBS (Welgene) and centrifuged at 500×g for 5 minutes at 4°C. The pellet was treated with 200µL TrypLE Express (Gibco) and digested at 37°C for 10 minutes, then digestion was stopped by adding 1mL cold DPBS containing 10% FBS (Welgene). Subsequently, centrifugation was performed under the same conditions as above to separate the pellet, which was washed with cold DPBS and centrifuged again. The obtained pellet was subcultured under the same conditions as above.

### 4. Produced Organoids

### (1) Production of Organoids

### ① Organoid Production

In the example (organoid production through 'freez-thaw dissociation'), organoids grew well and became large (see FIGS. 5 to 9), while in the comparative example (see FIG. 5), where organoid production was attempted through 'enzymatic dissociation', organoids were not well formed in both normal lung and lung cancer specimens, and it was not possible to confirm the various organoid characteristics as in the example.

### ② H&E Analysis of Normal Lung Organoids and Lung Cancer Organoids by Example

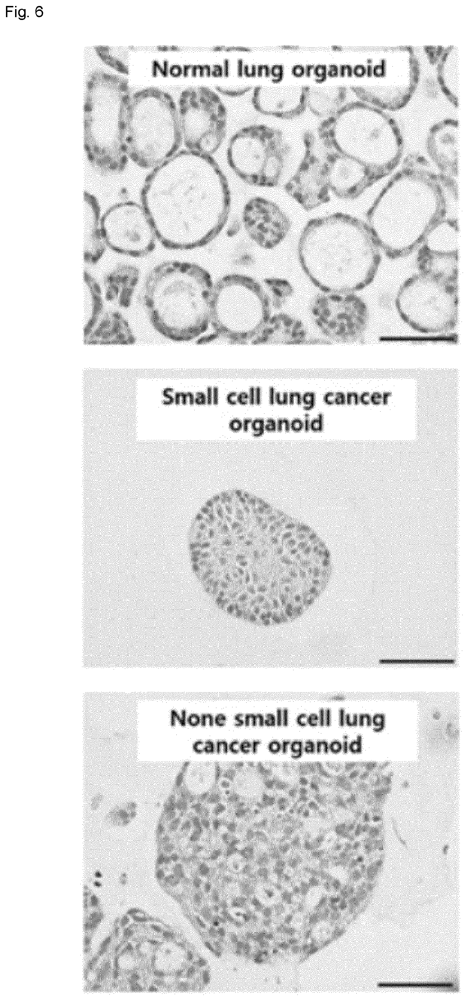

After 10 days in culture, the organoids of the example were stained with Hematoxylin and Eosin according to known methods (refer to Nat. Commun. 2019, 10, 3991) and analyzed by microscopy (see FIG. 6). In the photographs, Normal lung organoid was produced using cancer-free portions of resected lung, while small cell lung cancer and non-small cell lung cancer organoids were derived from small cell lung cancer and non-small cell lung cancer, respectively. In contrast, organoids did not form in either normal lung or lung cancer samples in the comparative example.

As shown in FIG. 6, normal lung organoids have the shape of normal airway cross-sections, whereas lung cancer organoids form compact clusters of various cells. Additionally, small cell lung cancer consists mainly of cells with large nuclei and little cytoplasm, while non-small cell lung cancer has smaller nuclei and more cytoplasm compared to small cell lung cancer. That is, from the photographs, normal lung organoids with structures similar to normal airway cross-sections and lung cancer organoids resembling small cell lung cancer and non-small cell lung cancer structures can be confirmed.

### ③ Bright Field Microscopy of Lung Organoids and Lung Cancer Organoids by Example

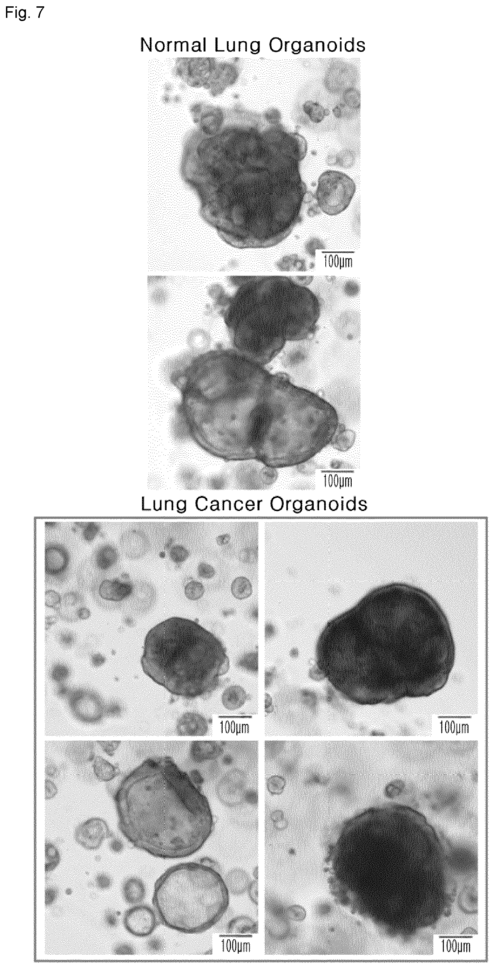

After 10 days of incubation, the organoids were observed under a light microscope (see FIG. 7). As shown in the photographs, organoids of substantial size were produced in all cases. No organoids were produced in the comparative example.

### ④ Holotomography of Lung Organoids and Lung Cancer Organoids by Example

Refractive index tomography was performed to confirm the three-dimensional structure of the organoids, the lumen structure at their center, and the surrounding cells.

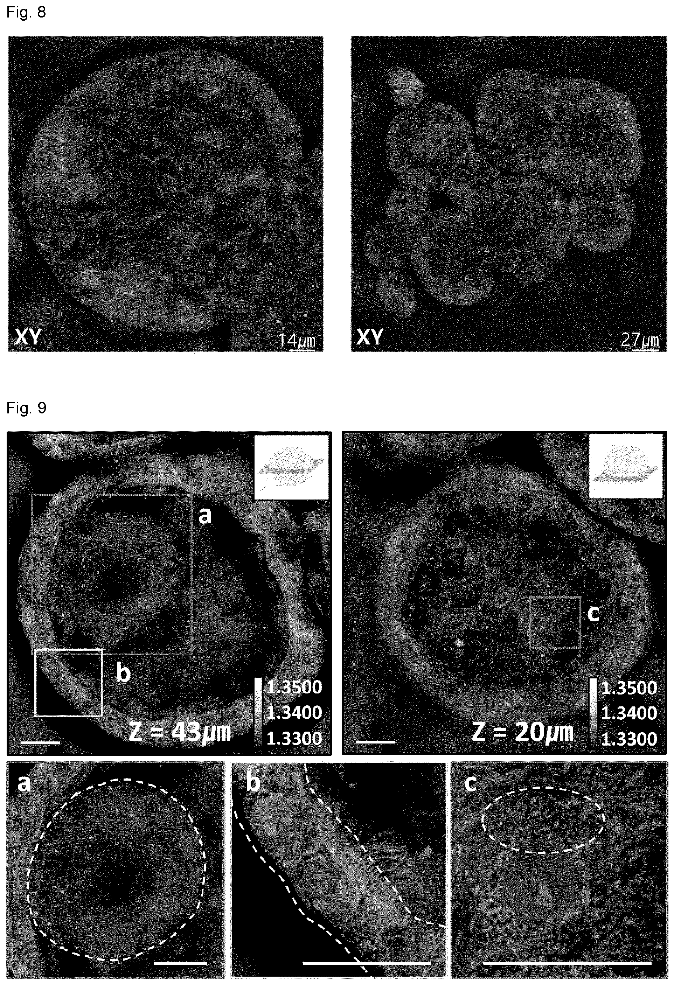

Holotomography was performed for non-small cell lung cancer organoids cultured for 10 days (see FIGS. 8, 9). Holotomography was performed as follows: Organoids were collected from the plate in cold DPBS (Welgene) and centrifuged at 500×g for 5 minutes at 4°C. The pellet was washed once with cold DPBS and centrifuged again (repeated three times). The organoid pellet was fixed in 4% paraformaldehyde (Biosesang) at 4°C for 24 hours, centrifuged at 500×g for 5 minutes at 4°C, washed once with DPBS, and centrifuged again (repeated two times). The organoid pellet in DPBS was transferred to a 50 mm imaging dish with a 1.5 bottom coverslip (TomoDish; Tomocube). Three-dimensional refractive index tomograms with a 140 µm Z scan range were acquired using HT-X1 holotomography (Tomocube) and visualized using image analysis software (TomoAnalysis version 1.5; Tomocube).

As shown in the figures, lung cancer organoids of various shapes composed of diverse cells were produced through the 'freeze-thaw dissociation' process according to the present invention (FIG. 8). Additionally, holotomography of the lung cancer organoids captured in multiple Z-sections (FIG. 9) shows (a) the mucosal layer, (b) apico-basal polarization, ciliated cells (red arrows), and (c) various intracellular organelles such as mitochondria and lipid droplets. A more detailed analysis reveals the following characteristics: Mucus secreted into the lumen was distinguished by a slightly higher refractive index distribution than airway mucus. Apico-basal polarization was observed in cells surrounding the clear lumen, and ciliated cells were clearly identified by their multiciliated structure. Subcellular structures of each cell, such as nucleus, nucleolus, mitochondria, and lipid droplets, were also observed.

Through these images, it was confirmed that the grown organoids reproduced the structure and function of human lungs in vivo. In contrast, as shown in FIG. 5, in the comparative example attempting organoid production through 'enzymatic dissociation', organoids did not form well in both normal lung and lung cancer specimens, making it impossible to confirm various organoid characteristics as in the example.

### (2) Analysis of Cancer Cell Purity in Organoids

Single-cell RNA sequencing was performed on normal lung organoids and lung cancer organoids (subcultured 2-3 times over 4-5 weeks) produced according to the exemple, following the method described in the prior art (Rev Physiol Biochem Pharmacol 2021;179:189-210), wherein the organoids were dissociated into single cells.

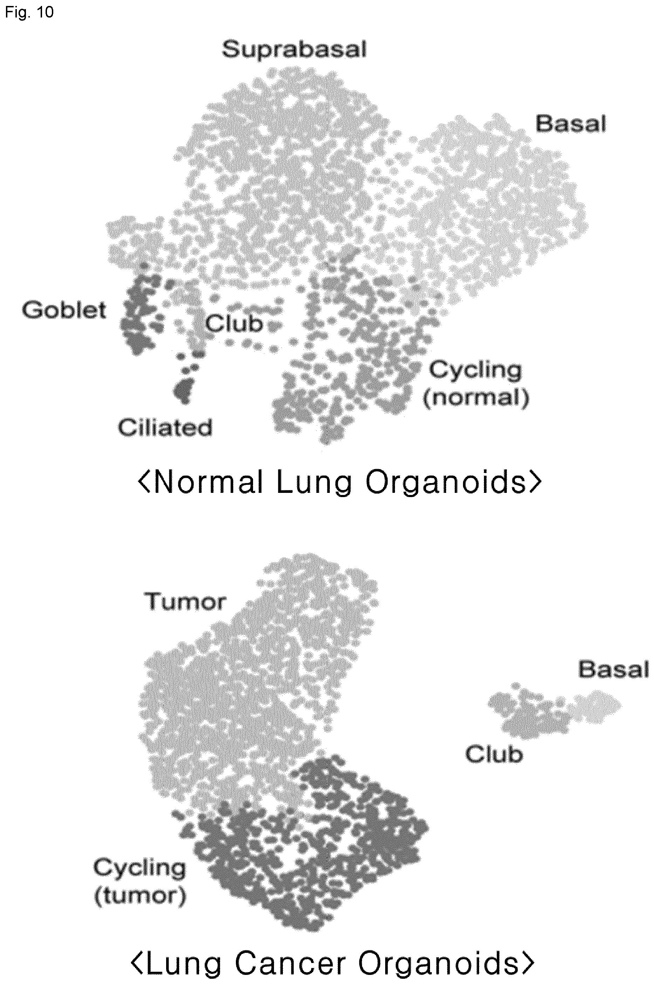

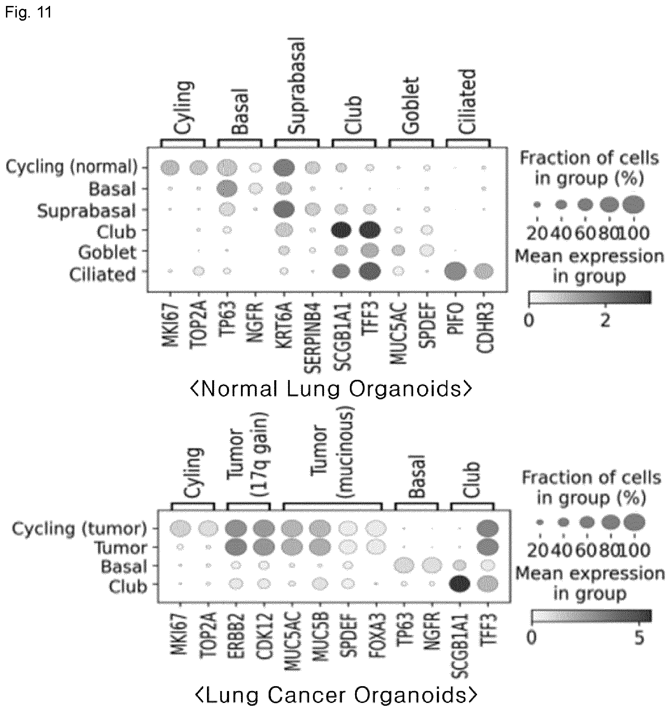

According to cell type annotation based on marker gene expression (Cancers 2021, 13, 3069), lung organoids comprise tumor cells (cancer cells), cycling cells (proliferating cells), and normal lung cells (basal cells, neuroendocrine cells, suprabasal cells, club cells, goblet cells, multiciliated cells, and deuterosomal cells). The single-cell RNA sequencing results and UMAP (Uniform Manifold Approximation and Projection; see Cell 2019, 177, 1888-1902.e21) of organoid cell types and states are shown in FIGS. 10 and 11, respectively.

As shown in the figures, the normal lung organoid example consists predominantly various normal lung cells with minimal cancer cells, while the lung cancer organoid of example consists mainly of tumor cells and proliferating cells. Thus, it was confirmed that the lung cancer organoids of the present invention comprise cancer cells of very high purity.

As shown in Figure 5, no organoids were produced in the comparative example.

### (3) Drug Sensitivity Analysis of Organoids

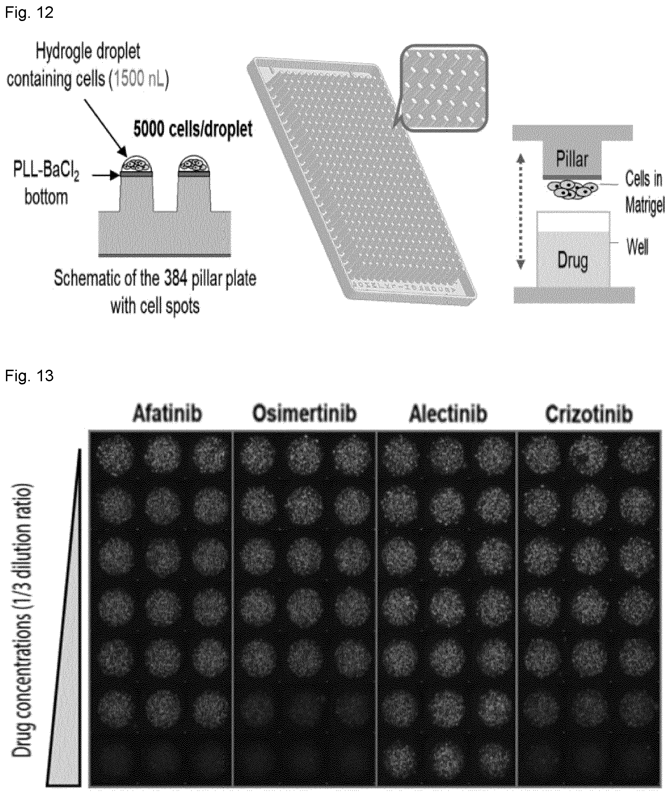

The lung cancer organoids comprising high-purity cancer cells obtained according to the present invention may be utilized for drug screening to determine the most effective drugs for treating lung cancer patients. To confirm this, as an exemple, a drug sensitivity test was conducted as follows using lung cancer organoids produced according to the method of the present invention ('freeze-thaw dissociation' to obtain cancer cells for organoid production) from lung cancer tissue exhibiting EGFR mutations. The experimental overview is conceptually illustrated in Figure 12.

The organoids were harvested and mixed with Matrigel (Corning Life Sciences, USA, catalog #354263) and seeded onto pillar plates at 5×10⁵ cells/mL. Next, the Matrigel-cell mixture (1.5 µL) was spotted onto the coated pillar plates using a precooled ASFA spotter (Medical & Bio Decision, Suwon, Republic of Korea) and incubated in an inverted position at 4°C for 15 minutes to allow cells to aggregate at the bottom of the spots. The plates were then placed in humidity chamber at 37°C to allow the spot to gel. Subsequently, the plates were combined with well plates containing fresh medium. After 3 days, cells were treated with EGFR or ALK targeting agents at six concentrations using the ASFA spotter. Cell viability was measured by staining with 0.5 µM calcein-AM at 37°C for 1 hour, followed by two PBS washes for 20 minutes. The organoid spots were scanned using an automated optical fluorescence scanner (ASFA scanner, Medical & Bio Decision, Korea). The scanner's microscope automatically focused on cell spots by moving in the z-direction, capturing 384 individual images at 4× magnification from one stained pillar/well plate. The images were then integrated into a single JPEG image for data analysis. The scanned images were analyzed using CellAnalyzer version 2.0 (Medical & Bio Decision). After scanning the cells, the organoid spots were transferred to new 384-well plates containing CellTiter-Glo 3D reagent to measure intracellular ATP levels via luminescence activity.

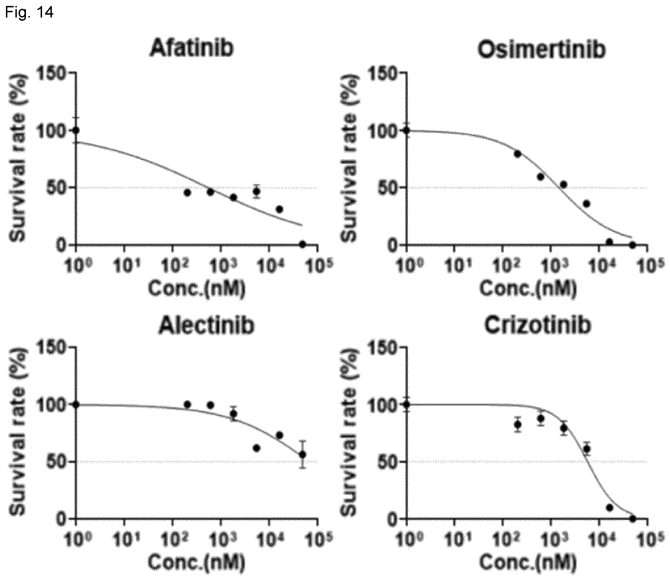

In brief, a total of 5000 single cells per pillar of the plate were seeded in 1.5µL medium and cultured for 3 days. Subsequently, various EGFR-TKIs and ALK inhibitors were administered at multiple doses for 3 days to determine IC50 and AUC (area under the curve). On day 6, organoid viability was confirmed using fluorescent dye Calcein-AM (see Figure 13), and survival rates for each drug were measured using CellTiter-Glo 3D cell viability assay (G9681; Promega) (see Figure 14).

The results confirmed that lung cancer organoids from EGFR-mutant lung cancer patients showed sensitivity to EGFR-TKIs (Afatinib, osimertinib) but not to ALK inhibitors (Alectinib, crizotinib), consistent with the clinical response of actual EGFR-mutant lung cancer patients (see table below)

| | IC₅₀ (µM) | AUC |
|---|---|---|
| Afatinib | 0.79 | 125.1 |
| Osimertinib | 1.8 | 133.9 |
| Alectinib | > 50 | 240.9 |
| Crizotinib | 7.1 | 177.2 |

These results suggest that cells obtained through 'freeze-thaw dissociation' proposed by the present invention produce organoids more effectively compared to cells obtained by conventional enzymatic methods, and that cancer cell organoids produced by the method of the present invention can be used for drug screening and personalized medicine.

## Claims

1. A method for producing organoids comprising:
(A) rapidly freezing biological tissue;
(B) thawing the rapidly frozen specimen;
(C) mechanically dissociating the thawed specimen to obtain dissociated cells; and
(D) producing organoids using the dissociated cells.

2. The method according to claim 1, wherein step (A) comprises:
a sub-step of contacting a cryoprobe tip to a predetermined site for obtaining dissociated cells from biological tissue for local freezing; and
a sub-step of isolating only the locally frozen region from the biological tissue.

3. The method according to claim 2, wherein the cryoprobe tip temperature is -40 to -130°C and the contact time is 3 to 5 seconds.

4. The method according to claim 2, wherein the biological tissue is isolated by biopsy.

5. The method according to claim 1, wherein the biological tissue is cancer tissue.

6. The method according to claim 5, wherein the biological tissue is lung cancer tissue.

7. The method according to claim 6, wherein the lung cancer tissue is obtained by non-surgical biopsy procedure.

8. The method according to claim 7, wherein the lung cancer tissue is obtained by non-surgical frozen biopsy procedure.
